# EUROPEAN PATENT APPLICATION

(11) **EP 3 125 143 A1**
(43) Date of publication of application: **01.02.2017**
(21) Application number: 15770242.4
(22) Date of filing: 20.03.2015
(51) Int. Cl.: G06F 19/22, C12Q 1/68

(54) **METHOD, DEVICE AND PROGRAM FOR GENERATING REFERENCE GENOME DATA, METHOD, DEVICE AND PROGRAM FOR GENERATING DIFFERENTIAL GENOME DATA, AND METHOD, DEVICE AND PROGRAM FOR RESTORING DATA**

(30) Priority: 24.03.2014 JP 2014060537
(71) Applicant: Kabushiki Kaisha Toshiba, Minato-ku Tokyo 105-8001 (JP)
(72) Inventor: MIYASHITA Shigeru, Tokyo 105-8001 (JP); SATOH Hajime, Tokyo 105-8001 (JP); IWATA Seiji, Tokyo 105-8001 (JP); TAKAYAMA Takuzo, Tokyo 105-8001 (JP)
(74) Representative: Noble, Nicholas
(86) International application number: PCT/JP2015/058577
(87) International publication number: WO 2015/146852

(57) **Abstract**

[Problem] The problem to be solved is to increase compression efficiency of genome data.

[Means for solving the problem] According to one embodiment, a method of generating reference genome data includes: determining, based on a plurality of base sequence data of different subjects, base information relating to at least one position in a base sequence according to a frequency of appearance of each of a plurality of base information at the position. The method further includes generating reference genome data to associate the determined base information with the position.

## Description

### FIELD

Embodiments of the present invention relate to a method, an apparatus, and a program for generating reference genome data, a method, an apparatus, and a program for generating difference genome data, and a method, an apparatus, and a program for recovering data.

### BACKGROUND

The human genome includes about 3.1 billion base pairs. The volume of genome data of one human being after full sequencing is several terabytes, and this is a large volume of data. At the site of genome research, the large-volume data is saved in a large storage device such as a hard disk.

Patent Literature 1: Japanese Patent Application Publication No. 2007-80034

When the genome data of several terabytes is saved, there is a problem that the cost of the storage device becomes enormous, and the cost of saving is too high. There is also a problem that when the data in terabytes is transmitted through a communication line, the cost of the line is too high, and it takes too long to communicate. However, an effect of reducing the volume of data is limited in a conventional data compression technique based on encoding similar to file compression.

The embodiments of the present invention are to provide a technique capable of improving a compression efficiency of genome data.

### SUMMARY

According to one embodiment, a method of generating reference genome data includes: determining, based on a plurality of base sequence data of different subjects, base information relating to at least one position in a base sequence according to a frequency of appearance of each of a plurality of base information at the position. The method further includes generating reference genome data to associate the determined base information with the position.

### BRIEF DESCRIPTION OF THE DRAWINGS

FIG. 1 is a diagram showing a configuration of a genome data transmission system S1 in a first embodiment;
FIG. 2 is a diagram showing a configuration of a data generation apparatus 4 in the first embodiment;
FIG. 3 is a diagram showing an example of difference genome data;
FIG. 4 is a diagram showing a configuration of a data recovery apparatus 6 in the first embodiment;
FIG. 5 is a flow chart showing an example of a process in the first embodiment;
FIG. 6 is a diagram showing a configuration of a genome data generation system GS3 in a third embodiment;
FIG. 7 is a flow chart showing an example of a generation process of reference genome data in the third embodiment;
FIG. 8 is a diagram showing a configuration of a genome data generation system GS4 in a fourth embodiment;
FIG. 9 is a flow chart showing an example of a generation process of reference genome data in the fourth embodiment;
FIG. 10 is a diagram showing a configuration of a genome data transmission system S5 in the fourth embodiment;
FIG. 11 is a diagram showing a configuration of a data generation apparatus 4e in the fourth embodiment;
FIG. 12 is an example of transmission data transmitted by a transmitter 44 according to the fourth embodiment;
FIG. 13 is a diagram showing a configuration of a data recovery apparatus 6e in the fourth embodiment;
FIG. 14 is a flow chart showing an example of a generation process of reference genome data in the fourth embodiment; and
FIGS. 15A and 15B each are a flow chart showing a specific example of the generation process of the reference genome data in the fourth embodiment.

### DETAILED DESCRIPTION

Embodiments of the present invention will now be described with reference to the drawings. Most of 3.1 billion base pairs in human genome are common to individuals, and single nucleotide polymorphisms (SNP) indicating individual differences account for about 0.1% between two arbitrary individuals. Therefore, reference genome data serving as a reference of genome data is set in advance in each embodiment, and only base information that varies between the genome data of each person and the reference genome data is saved and transmitted. The genome data here is a kind of base sequence data indicating genetic information and is entire base sequence data of a human individual.

As a result, the genome data of each person can be compressed to a volume of data that is about 0.1% of normal genome data. A data generation apparatus on a transmission side configured to transmit the genome data and a data recovery apparatus on a reception side can store common reference genome data in advance, and the data recovery apparatus can recover full genome data based on the received data and the reference genome data.

### (First Embodiment)

A genome data transmission system of a first embodiment sets the genome data decoded and publicized by the international human genome project as the reference genome data. The genome data transmission system compares the reference genome data and subject genome data (genome data actually measured from a person being tested or human subject by survey) at each SNP position. The genome data transmission system generates difference genome data obtained by extracting base information of the subject genome data at base sequence positions where the base information of the subject genome data at corresponding base sequence positions is different from the base information of the reference genome data.

The genome data transmission system transmits the generated difference genome data and substitutes the received difference genome data for data of part of the reference genome data at a transmission destination to thereby recover the data. The generation of the difference genome data is a kind of lossless compression, because the original subject genome data can be recovered based on the difference genome data.

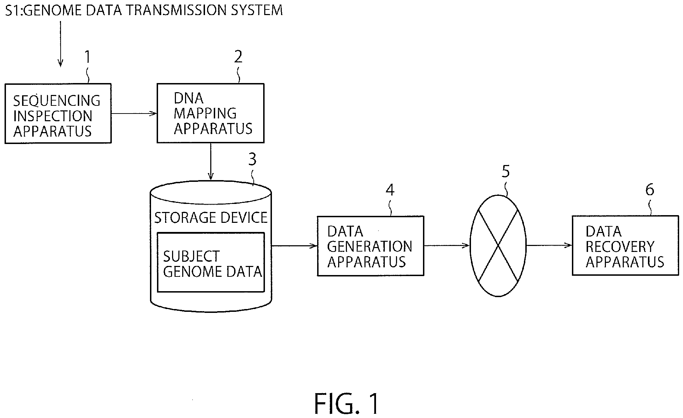

A configuration of a genome data transmission system S1 in the first embodiment will be described with reference to FIG. 1. Fig. 1 is a diagram showing a configuration of the genome data transmission system S1 in the first embodiment. As shown in FIG. 1, the genome data transmission system S1 includes: a sequencing inspection apparatus 1; a DNA mapping apparatus 2 connected to the sequencing inspection apparatus 1 by wiring; a storage device 3 connected to the DNA mapping apparatus 2 by wiring; a data generation apparatus 4 connected to the storage device 3 by wiring; a communication circuit network 5; and a data recovery apparatus 6 connected to the data generation apparatus 4 by wiring through the communication circuit network 5.

The sequencing inspection apparatus 1 applies DNA (deoxyribonucleic acid) sequencing to a target sample obtained by biochemically processing blood of a specific subject (person being tested or human subjected). The sequencing inspection apparatus 1 outputs a plurality of fragmented base sequence data obtained by the DNA sequencing to the DNA mapping apparatus 2.

The DNA mapping apparatus 2 collates the plurality of fragmented base sequence data with reference human genome sequence data and saves subject genome data obtained by the collation in the storage device 3. The subject genome data here is base sequence data of a specific subject. As a result, the subject genome data is saved in the storage device 3. The subject genome data here is a set of position information indicating positions of bases and base information indicating bases of DNA. There are four types of bases of DNA: adenine (A), thymine (T), guanine (G), and cytosine (C). The base information indicating the base of DNA is, for example, a base symbol (one of A, T, G, and C).

The data generation apparatus 4 reads the subject genome data from the storage device 3 and compares the reference genome data set in advance and the subject genome data at each SNP position. The data generation apparatus 4 generates difference genome data obtained by extracting the base information of the subject genome data at base sequence positions where values vary between the reference genome data and the subject genome data. The difference genome data here is data including a set of position information indicating the base sequence positions of DNA and base information indicating the bases of DNA.

The data recovery apparatus 6 substitutes the base information of the difference genome data for the base information of the reference genome data at the base sequence positions indicated by the position information included in the difference genome data. The data recovery apparatus 6 applies this process to the data at all positions of the bases of DNA included in the difference genome data. As a result, a subject genome data set is recovered.

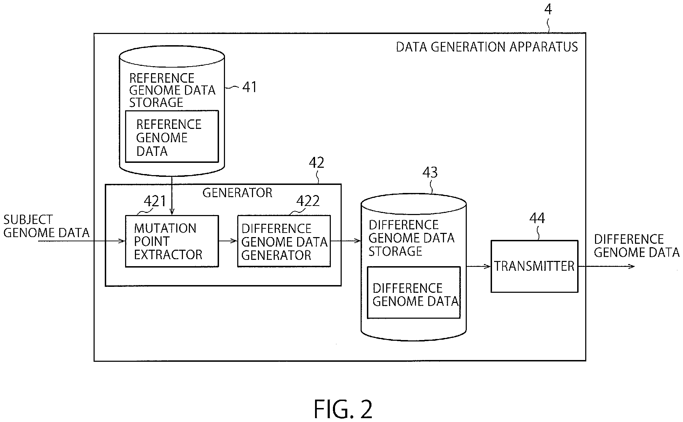

Subsequently, a configuration of the data generation apparatus 4 in the first embodiment will be described with reference to FIG. 2. FIG. 2 is a diagram showing a configuration of the data generation apparatus 4 in the first embodiment. As shown in FIG. 2, the data generation apparatus 4 includes a reference genome data storage 41, a generator 42, a difference genome data storage 43, and a transmitter 44.

Reference genome data is saved in the reference genome data storage 41. An example of the reference genome data in the present embodiment includes a genome data set decoded and publicized by the international human genome project. The reference genome data is data including a plurality of sets of position information indicating positions of bases of DNA and base information indicating the bases of DNA.

The generator 42 compares the base sequence data of a specific subject and the reference genome data set in advance. The generator 42 then generates difference genome data including a plurality of sets of position information indicating base sequence positions with different base information and base information at the base sequence positions indicated in the position information in the base sequence data of the specific subject. Specifically, for example, the generator 42 extracts base sequence positions where the base information of the subject genome data at corresponding base sequence positions is different from the base information of the reference genome data and generates difference genome data including a plurality of sets of the position information of the extracted base sequence positions and the base information at the base sequence positions in the subject genome data.

The generator 42 is formed by, for example, a device configured to perform electronic control. The device configured to perform electronic control includes, for example, a CPU (Central Processing Unit), a ROM (Read Only Memory) storing a program, and a RAM (Random Access Memory) for primary storage of data. The CPU reads out the program stored in the ROM to the RAM and executes the program to function as the generator 42. The generator 42 here includes a mutation point extractor 421 and a difference genome data generator 422.

The mutation point extractor 421 reads the subject genome data from the storage device 3. The mutation point extractor 421 also reads the reference genome data from the reference genome data storage 41. The mutation point extractor 421 compares the subject genome data and the reference genome data and extracts mutation points that are base sequence positions with different base information at corresponding base sequence positions. The mutation point extractor 421 transfers information indicating the extracted mutation points to the difference genome data generator 422.

The difference genome data generator 422 generates difference genome data including a plurality of sets of mutation point information indicating the mutation points extracted by the mutation point extractor 421 and the base information at the mutation points in the subject genome data. The generated difference genome data is saved in the difference genome data storage 43. As a result, the difference genome data is saved in the difference genome data storage 43.

The difference genome data is input to the transmitter 44 from the difference genome data storage 43, and the transmitter 44 transmits the input difference genome data to the data recovery apparatus 6 through the communication circuit network 5.

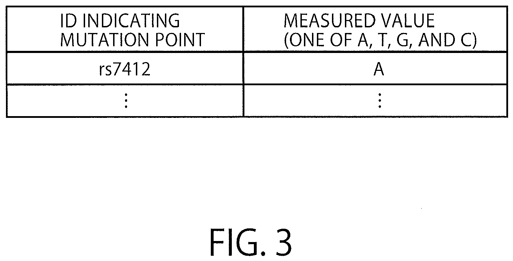

Subsequently, the difference genome data will be described with reference to FIG. 3. FIG. 3 is a diagram showing an example of the difference genome data. The difference genome data shown in FIG. 3 is data including a plurality of sets of IDs indicating the mutation points and measured values. The IDs indicating the mutation points in FIG. 3 are indicated by, for example, rs numbers (Reference SNP ID numbers). The measured value is a measured base symbol (one of A, T, G, and C).

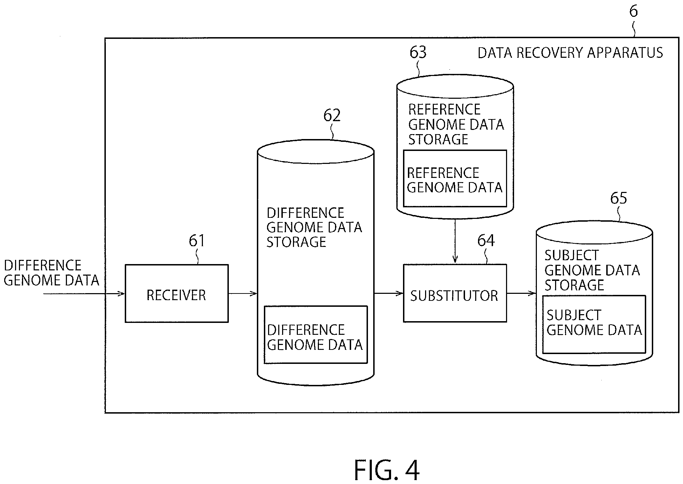

Subsequently, a configuration of the data recovery apparatus 6 will be described with reference to FIG. 4. FIG. 4 is a diagram showing a configuration of the data recovery apparatus 6 in the first embodiment. As shown in FIG. 4, the data recovery apparatus 6 includes a receiver 61, a difference genome data storage 62, a reference genome data storage 63, a substitutor 64, and a subject genome data storage 65.

The receiver 61 receives the difference genome data transmitted by the data generation apparatus 4 through the communication circuit network 5 and outputs the received difference genome data to the difference genome data storage 62.

The difference genome data storage 62 saves the difference genome data input from the receiver 61.

The same reference genome data as the reference genome data stored in the reference genome data storage 41 of the data generation apparatus 4 is saved in the reference genome data storage 63.

The substitutor 64 reads the difference genome data from the difference genome data storage 62. The substitutor 64 also reads the reference genome data from the reference genome data storage 63. The substitutor 64 substitutes the base information corresponding to the mutation points in the difference genome data for the base information at the mutation points among the bases included in the reference genome data. The substitutor 64 applies the substitution process to all mutation points to recover the subject genome data. The substitutor 64 saves the recovered subject genome data in the subject genome data storage 65.

The substitutor 64 here is formed by, for example, a device configured to perform electronic control. The device configured to perform electronic control includes, for example, a CPU, a ROM saving a program, and a RAM for primary storage of data. The CPU reads out the program saved in the ROM to the RAM and executes the program to function as the substitutor 64.

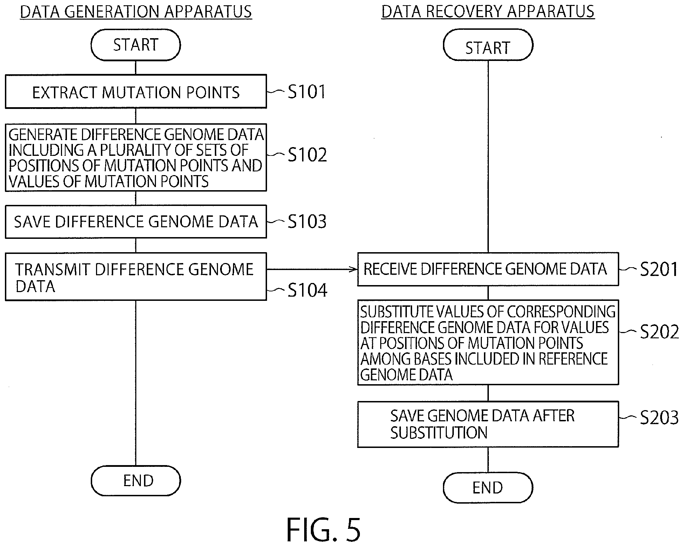

Subsequently, a flow of a process of the genome data transmission system S1 in the first embodiment will be described with reference to FIG. 5. FIG. 5 is a flow chart showing an example of the process in the first embodiment. It is assumed here that the subject genome data is already saved in the storage device 3.

(Step S101) The mutation point extractor 421 compares the subject genome data and the reference genome data and extracts the mutation points that are base sequence positions with different base information at corresponding base sequence positions.

(Step S102) The difference genome data generator 422 generates difference genome data including sets of the mutation points and the base information at the mutation points in the subject genome data.

(Step S103) The difference genome data generator 422 saves the generated difference genome data in the difference genome data storage 43.

(Step S104) The transmitter 44 transmits the difference genome data to the data recovery apparatus 6 through the communication circuit network 5.

(Step S201) The receiver 61 receives the difference genome data transmitted by the transmitter 44 in step S104.

(Step S202) The substitutor 64 substitutes the base information at the mutation points in the difference genome data for the base information at the mutation points among the bases included in the reference genome data.

(Step S203) The substitutor 64 saves the substituted and recovered subject genome data in the subject genome data storage 65.

As described, the data generation apparatus 4 compares the subject genome data and the reference genome data in the genome data transmission system S1 according to the first embodiment. The data generation apparatus 4 extracts the mutation points where the base information of the subject genome data at corresponding base sequence positions is different from the base information of the reference genome data and generates the difference genome data including a plurality of sets of the extracted mutation points and the base information at the mutation points in the subject genome data. The data recovery apparatus 6 substitutes the bases at the mutation points in the difference genome data for the bases at the mutation points among the bases included in the reference genome data to recover the subject genome data.

Therefore, the data generation apparatus 4 can convert the subject genome data with a data volume in terabytes into the difference genome data in gigabytes that is about 1/1000, and the cost of saving the genome data of each subject can be reduced. The volume of the difference genome data is in gigabytes, and data transmission using a general communication line can be realized in realistic time and cost. More specifically, the cost of the line can be reduced, and the communication time can be reduced. The data recovery apparatus 6 can recover the subject genome data without losing the information included in the subject genome data.

The publicized genome data set used as the reference genome data in the present embodiment is not personal information, and confidentiality is not necessary. Therefore, the data does not have to be managed by concealing the data, and the management cost can be reduced.

### (Second Embodiment)

Subsequently, a second embodiment will be described. The genome data set decoded and publicized by the international human genome project is used as the reference genome data in the first embodiment. The second embodiment is different in that actual genome data measured from a representative subject representing a specific haplotype or region is used as the reference genome data, and a difference between the reference genome data and genome data of a subject belonging to the same haplotype or region as that of the representative subject is obtained.

A population with a same haplotype has common ancestors with same or similar SNP mutation points. For example, people living in a large number in Japan (hereinafter, called "Japanese people") are a population with the same haplotype, and the population will be called a haplogroup of Japanese people. Similarly, people living in a large number in Tibet (hereinafter, called "Tibetan people") are a population with the same haplotype, and the population will be called a haplogroup of Tibetan people. In the second embodiment, genome data measured from a representative subject representing the haplotype with different genetic features is used as the reference genome data, for example.

The genome data transmission system S1 in the second embodiment is different from the genome data transmission system S1 in the first embodiment in that the reference genome data stored in the reference genome data storages 41 and 63 is the genome data measured from the representative subject representing a specific haplotype or region.

The sequencing inspection apparatus 1 applies DNA sequencing to a target sample obtained by biochemically processing blood of a subject belonging to the same haplotype or region as that of the representative subject. As a result, the subject genome data of the subject belonging to the same haplotype or region as that of the representative subject is saved in the storage device 3.

The other configuration is the same as the configuration of the genome data transmission system S1 in the first embodiment, and the description of the configuration of the genome data transmission system S1 in the second embodiment will not be repeated.

As described, the genome data measured from the representative subject representing a specific haplotype or region is used as the reference genome data in the genome data transmission system S1 according to the second embodiment. The subject is a subject belonging to the same haplotype or region as that of the representative subject. Therefore, the difference between the bases of the reference genome data and the bases of the subject genome data can be small, and the number of mutation points can be reduced. As a result, the volume of the difference genome data can be further reduced.

### (Third Embodiment)

Subsequently, a third embodiment will be described. In the second embodiment, the actual genome data measured from the representative subject representing a specific haplotype or region is used as the reference genome data, and the difference between the reference genome data and the genome data of the subject belonging to the same haplotype or region as that of the representative subject is obtained.

In the third embodiment, a plurality of genome data of a specific haplotype or region (for example, Japan) are analyzed, and data of a most frequent value at each SNP position is generated as reference genome data. A difference between the reference genome data and genome data of a subject belonging to the same specific haplotype or region as that of the specific haplotype or region used in creating the reference genome data is obtained.

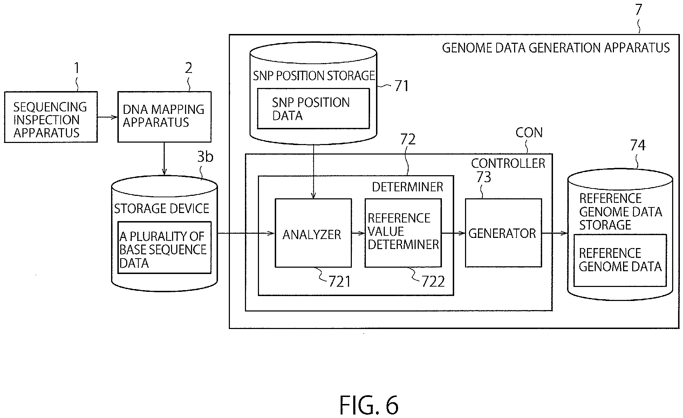

Subsequently, a configuration of a genome data generation system GS3 in the third embodiment will be described with reference to FIG. 6. FIG. 6 is a diagram showing a configuration of the genome data generation system GS3 in the third embodiment. As shown in FIG. 6, the genome data generation system GS3 includes the sequencing inspection apparatus 1, the DNA mapping apparatus 2, a storage device 3b, and a genome data generation apparatus 7. The same reference signs are provided to the same constituent elements as in FIG. 1, and the description will not be repeated.

Subsequently, operation of the genome data generation system GS3 in the third embodiment will be described.

In the genome data generation system GS3, the sequencing inspection apparatus 1 applies DNA sequencing to samples obtained from a plurality of subjects in a specific haplotype or region (for example, Japan). Every time the sequencing inspection apparatus 1 obtains a plurality of fragmented base sequence data of a subject, the DNA mapping apparatus 2 sequentially collates the plurality of fragmented base sequence data with reference human genome sequence data. The DNA mapping apparatus 2 saves the base sequence data obtained by the collation in the storage device 3b. As a result, the base sequence data of a plurality of subjects is saved in the storage device 3b. The genome data generation apparatus 7 then generates reference genome data based on the base sequence data of a plurality of subjects.

Subsequently, a configuration of the genome data generation apparatus 7 will be described with reference to FIG. 6. As shown in FIG. 6, the genome data generation apparatus 7 includes an SNP position storage 71, a controller CON, and a reference genome data storage 74.

SNP position data indicating each SNP position is stored in the SNP position storage 71.

The controller CON controls the constituent elements included in the genome data generation apparatus 7. The controller CON is formed by, for example, a device configured to perform electronic control. The device configured to perform electronic control includes, for example, a CPU (Central Processing Unit), a ROM (Read Only Memory) storing a program, and a RAM (Random Access Memory) for primary storage of data. The CPU reads out the program stored in the ROM to the RAM and executes the program to function as the controller CON. The controller CON includes a determiner 72 and a generator 73.

The determiner 72 determines base information in at least one position of the base sequence according to a frequency of appearance of the base information at the position in a plurality of base sequence data with different subjects. It is preferable that the at least one position here is at least one position of SNP.

In the description of the present embodiment, the at least one position is, for example, a position of each SNP. Based on this, for each position of SNP, the determiner 72 determines the base information with the highest frequency of appearance in the plurality of base sequence data as the base information at the position of SNP, for example.

The determiner 72 here includes an analyzer 721 and a reference value determiner 722.

The analyzer 721 counts the frequency of appearance of each base (i.e. A, T, G, and C) of DNA at each SNP position in the plurality of base sequence data.

The reference value determiner 722 uses the frequency of appearance obtained by the analyzer 721 to determine the base information with the highest frequency of appearance in each SNP position as the base information (hereinafter, also called "reference value") of the corresponding position in the reference genome data.

The generator 73 generates reference genome data associating the base information determined by the determiner 72 with the positions. Specifically, the generator 73 includes the plurality of base information determined by the determiner 72 in the reference genome data in association with the positions of SNP, for example. The generator 73 further includes base information at positions other than the positions of SNP extracted from at least one of the plurality of base sequence data in the reference genome data in association with the positions other than the positions of SNP, for example.

The values at the positions other than the positions of SNP are values common to the base sequence data of a plurality of subjects. The generator 73 stores the generated reference genome data in the reference genome data storage 74.

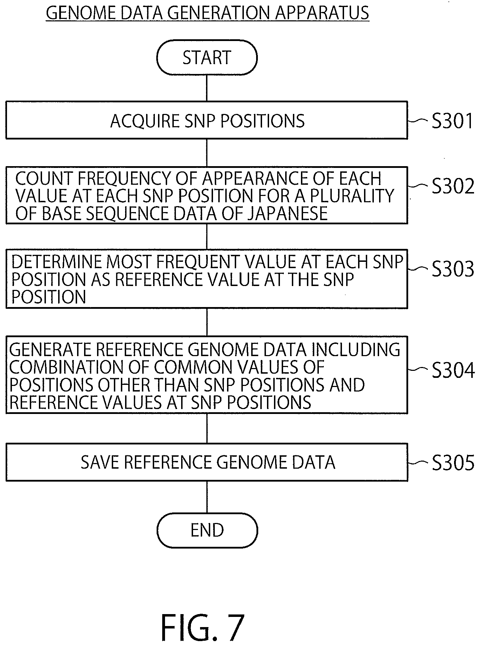

Subsequently, a generation process of the reference genome data in the third embodiment will be described with reference to FIG. 7. FIG. 7 is a flow chart showing an example of a generation process of the reference genome data in the third embodiment. It is assumed here that the base sequence data of a plurality of Japanese subjects are already saved in the storage device 3b, for example.

(Step S301) The analyzer 721 reads the SNP position data to acquire each SNP position.

(Step S302) The analyzer 721 reads the base sequence data of a plurality of subjects from the storage device 3, for example. The plurality of base sequence data are, for example, a plurality of base sequence data of Japanese. The analyzer 721 counts the frequency of appearance of each base information (for example, base symbol) at each SNP position for the plurality of read base sequence data.

(Step S303) The reference value determiner 722 determines the base information with the highest frequency of appearance at each SNP position as the reference value at the position.

(Step S304) The generator 73 includes each reference value determined in step S303 in the reference genome data in association with the corresponding SNP position. The generator 73 also includes the base information at positions other than the SNP positions extracted from at least one of the base sequence data of a plurality of subjects in the reference genome data in association with the positions other than the SNP positions.

(Step S305) The generator 73 stores the generated reference genome data in the reference genome data storage 74.

Subsequently, a configuration of the genome data transmission system S1 according to the third embodiment will be described. The genome data transmission system S1 in the third embodiment is different from the genome data transmission system S1 in the first embodiment in that the reference genome data stored in the reference genome data storages 41 and 63 is reference genome data of a specific haplotype or region generated by the generator 73 according to the third embodiment. The other configuration is the same as in the genome data transmission system S1 in the first embodiment, and the description of the configuration of the genome data transmission system S1 according to the third embodiment will not be repeated.

Subsequently, operation of the genome data transmission system S1 according to the third embodiment will be described. The sequencing inspection apparatus 1 according to the third embodiment applies DNA sequencing to target samples obtained by biochemically processing the blood of the subjects belonging to the same haplotype or region as the haplotype or region selected in generating the reference genome data. As a result, the subject genome data of the subjects belonging to the same haplotype or region as the haplotype or region selected in generating the reference genome data is saved in the storage device 3. This is different from the first embodiment.

The subsequent flow of the process by the genome data transmission system S1 according in the third embodiment is the same as the process by the genome data transmission system S1 in the first embodiment illustrated in FIG. 5, and the description will not be repeated.

As described, the genome data generation apparatus 7 according to the third embodiment determines the base information with the highest frequency of appearance at each SNP position as the reference value in the base sequence data of a plurality of subjects belonging to a specific haplotype or region. The genome data generation apparatus 7 includes each reference value in the reference genome data in association with the corresponding SNP position. The generator 73 further includes the base information at the positions other than the SNP positions extracted from at least one of the plurality of base sequence data in the reference genome data in association with the positions other than the SNP positions.

As a result, the difference between the reference genome data and the subject genome data of the subjects belonging to the specific haplotype or region can be smaller than in the second embodiment, and the volume of the difference genome data can be smaller than in the second embodiment.

The reference genome data of the present embodiment is not personal information, and confidentiality is not necessary. As in the second embodiment, the data does not have to be managed by concealing the data, and the management cost can be reduced.

### (Fourth Embodiment)

Subsequently, a fourth embodiment will be described. In the third embodiment, a plurality of genome data of a specific haplotype or region (for example, Japan) are analyzed, and the data with the most frequent value at each SNP position is generated as the reference genome data.

In the fourth embodiment, the genome data is classified into a plurality of attribute classes with different genetic features based on one or a combination of specific haplotype, region, gender, and blood type, and the reference genome data is generated for each attribute class.

For example, when the genome data is classified based on the gender, the plurality of attribute classes include two classes, a class with a haplotype common to male and a class with a haplotype common to female. The reference genome data is generated for each class. When the genome data is classified based on the blood type for example, the plurality of attribute classes include classes with haplotypes common to type A, type B, type O, and type AB, and the reference genome data is generated for each class. The attribute classes may include classes with haplotypes common to Rh+ and Rh-.

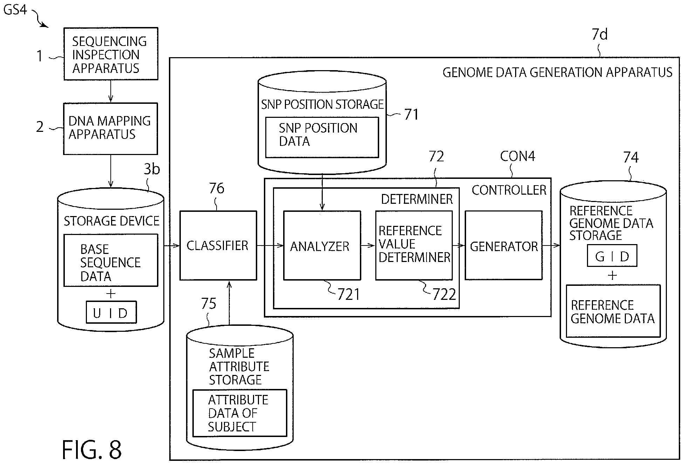

Subsequently, a configuration of a genome data generation system GS4 in the fourth embodiment will be described. FIG. 8 is a diagram showing a configuration of the genome data generation system GS4 in the fourth embodiment. Compared to the configuration of the genome data generation system GS3 in the third embodiment, the controller CON is changed to a controller CON4, and a sample attribute storage 75 is added in the configuration of the genome data generation system GS4 in the fourth embodiment in FIG. 8.

Attribute data of subjects is stored in the sample attribute storage 75. The attribute data of subjects is, for example, data in which sets of subject identification information UID for identifying the subjects, haplotypes, gender, blood types, and the like are collected for the subjects.

Compared to the configuration of the controller CON according to the third embodiment, a classifier 76 is added in the configuration of the controller CON4 according to the fourth embodiment.

A plurality of base sequence data are stored in the storage device 3b, and each base sequence data is stored in association with subject identification information for identifying the subject from which the base sequence data is extracted.

The classifier 76 classifies the plurality of base sequence data into the attributes of the subjects from which the base sequence data are extracted. Specifically, the classifier 76 reads a set of the base sequence data and the subject identification information from the storage device 3b, for example. The classifier 76 reads, from the sample attribute storage 75, the attribute of the subject corresponding to the subject identification information read from the storage device 3b. The classifier 76 uses the read attribute of the subject to classify the base sequence data read from the storage device 3b into one of the plurality of attribute classes. The classifier 76 repeats the process for each base sequence data. As a result, the base sequence data are classified into the attribute classes of the subjects.

For each attribute class, the determiner 72 determines the base information with the highest frequency of appearance at each position of SNP in the plurality of base sequence data classified into the attribute class.

Specifically, the determiner 72 uses the plurality of base sequence data classified into the target attribute class to count the frequency of appearance of the base information at each position of SNP, for example. The determiner 72 uses the result of counting to determine the base information (for example, base symbol) with the highest frequency of appearance at each position of SNP. The determiner 72 applies the process to all attribute classes.

For each attribute class, the generator 73 generates reference genome data including the base information with the highest frequency of appearance at each position of SNP determined for each attribute class and including the base information at the positions other than the SNP extracted from the plurality of base sequence data. The generator 73 stores each of the generated reference genome data in the reference genome data storage 74 in association with reference genome data identification information GID for identifying the corresponding reference genome data.

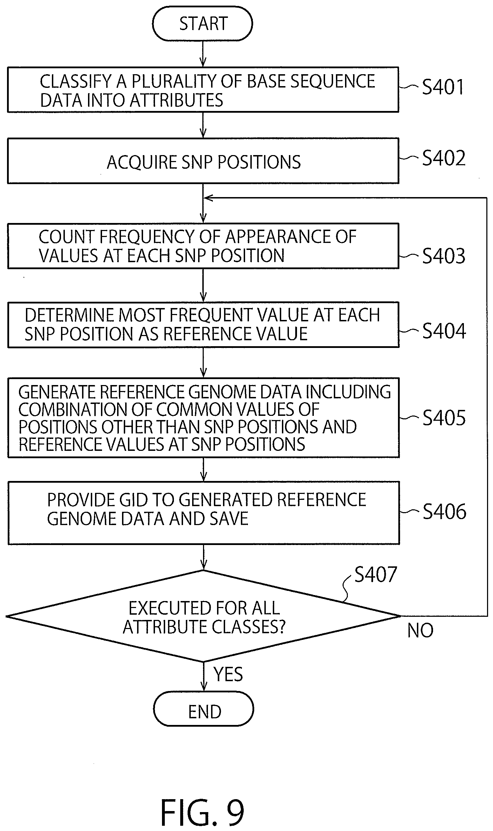

A generation process of the reference genome data in the fourth embodiment will be described with reference to FIG. 9. FIG. 9 is a flow chart showing an example of a generation process of the reference genome data in the fourth embodiment. It is assumed here that a plurality of base sequence data are already saved in the storage device 3b in association with the subject identification information UID, for example.

(Step S401) The classifier 76 classifies the base sequence data into one of the plurality of attribute classes according to the attribute every time a set of the base sequence data and the subject identification information UID is read. For example, when the base sequence data is classified based on a set of gender and blood type, the attribute classes include eight attribute classes: male and type A, female and type A, male and type B, female and type B, male and type O, female and type O, male and type AB, and female and type AB. Therefore, the classifier 76 classifies the base sequence data into one of the eight attribute classes, for example.

(Step S402) The analyzer 721 reads the SNP position data from the SNP position storage 71 to acquire the SNP positions.

(Step S403) The analyzer 721 counts the frequency of appearance of the base information at each SNP position for the target attribute class.

(Step S404) The reference value determiner 722 uses the frequency of appearance counted in step S403 to determine the base information with the highest frequency of appearance at the SNP position as the reference value.

(Step S405) For the target attribute class, the generator 73 generates reference genome data including the base information common to the plurality of base sequence data at the positions other than the SNP positions and including the reference values at the SNP positions determined in step S404.

(Step S406) The generator 73 stores the generated reference genome data in the reference genome data storage 74 in association with the reference genome data identification information GID for identifying the reference genome data.

(Step S407) The controller CON determines whether the process of steps S403 to S406 is executed for all attribute classes. If the process of steps S403 to S406 is not executed for all attribute classes (NO), the controller CON returns to step S403 and continues the process for the next attribute class. On the other hand, if the process of steps S403 to S406 is executed for all attribute classes (YES), the controller CON ends the process.

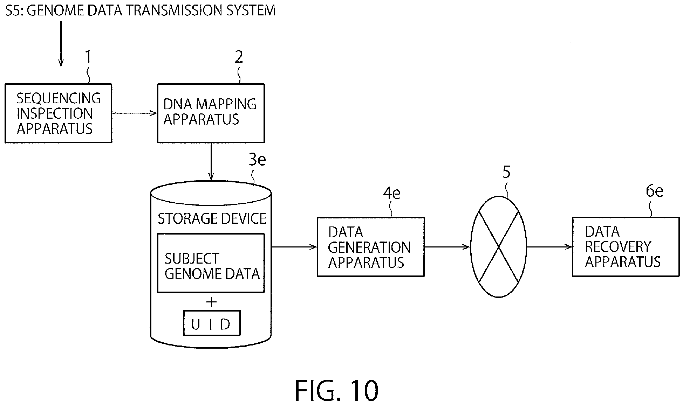

Subsequently, a configuration of a genome data transmission system S5 in the fourth embodiment will be described with reference to FIG. 10. FIG. 10 is a diagram showing a configuration of the genome data transmission system S5 in the fourth embodiment. Compared to the configuration of the genome data transmission system S1 in the first embodiment (see FIG. 1), the storage device 3 is changed to a storage device 3e, the data generation apparatus 4 is changed to a data generation apparatus 4e, and the data recovery apparatus 6 is changed to a data recovery apparatus 6e in the configuration of the genome data transmission system S5 in the fourth embodiment. The same reference signs are provided to the same constituent elements as in FIG. 1, and the description will not be repeated.

It is assumed that the subject identification information UID for identifying the subject corresponding to the subject genome data is associated and stored in the storage device 3e.

N (N is an integer 2 or greater) reference genome data Gs1, ..., GsN generated for each attribute class by a genome data generation apparatus 7d are stored in the data generation apparatus 4e and the data recovery apparatus 6e.

The data generation apparatus 4e generates difference genome data by using the reference genome data according to the attribute class that the subject belongs to and transmits the difference genome data to the data recovery apparatus 6e.

The data recovery apparatus 6e uses the same reference genome data as the reference genome data used by the data generation apparatus 4e and recovers the subject genome data based on the difference genome data received from the data generation apparatus 4e.

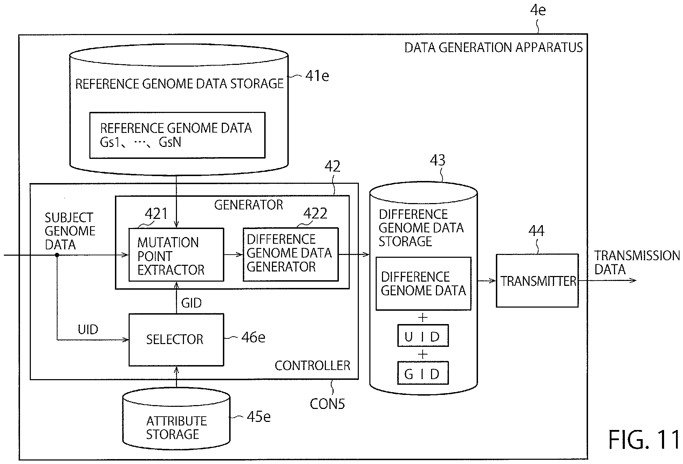

Subsequently, a configuration of the data generation apparatus 4e in the fourth embodiment will be described with reference to FIG. 11. FIG. 11 is a diagram showing a configuration of the data generation apparatus 4e in the fourth embodiment. The data generation apparatus 4e includes a reference genome data storage 41e, a controller CON5, the difference genome data storage 43, the transmitter 44, and an attribute storage 45e. The same reference signs are provided to the same constituent elements as in FIG. 2, and the description will not be repeated.

The controller CON5 is formed by, for example, a device configured to perform electronic control. The device configured to perform electronic control includes, for example, a CPU, a ROM storing a program, and a RAM for primary storage of data. The CPU reads out the program stored in the ROM to the RAM and executes the program to function as the controller CON5. The controller CON5 includes the generator 42 and a selector 46e.

The N reference genome data Gs1, ..., GsN generated for each attribute class by the genome data generation apparatus 7d are stored in the reference genome data storage 41e.

The attribute data of each subject is stored in the attribute storage 45e. The attribute data of each subject here is data associating the subject identification information UID and the attribute information indicating the corresponding attribute of the subject.

The selector 46e selects the reference genome data of the attribute that a specific subject belongs to from the plurality of reference genome data provided for each attribute class of the subject. Specifically, the selector 46e reads the subject identification information UID from the storage device 3e and reads the attribute information corresponding to the read subject identification information UID from the attribute storage 45e. The selector 46e selects the reference genome data identification information GID corresponding to the read attribute information and outputs the selected reference genome data identification information GID to the mutation point extractor 421.

The mutation point extractor 421 reads, from the reference genome data storage 41e, the reference genome data specified by the reference genome data identification information GID input from the selector 46e. The mutation point extractor 421 also reads the subject genome data from the storage device 3e. The mutation point extractor 421 compares the reference genome data and the subject genome data and extracts mutation points where the base information varies between corresponding base sequence positions.

The difference genome data generator 422 generates difference genome data including a plurality of sets of the mutation points and the base information at the mutation points in the subject genome data. The difference genome data generator 422 associates and stores, in the difference genome data storage 43, the subject identification information UID, the reference genome data identification information GID, and the generated difference genome data.

The transmitter 44 transmits transmission data including the subject identification information UID and the reference genome data identification information GID in addition to the difference genome data to the data recovery apparatus 6e.

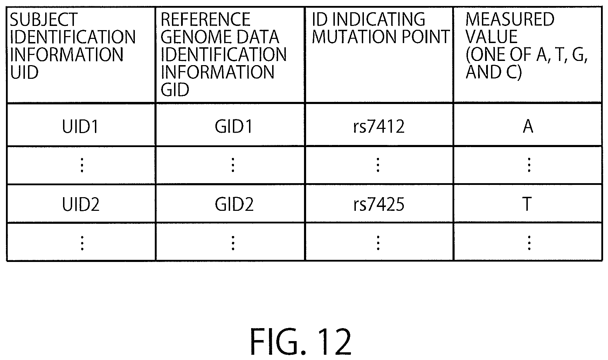

FIG. 12 is an example of the transmission data transmitted by the transmitter 44 according to the fourth embodiment. As shown in FIG. 12, the transmission data is data including a plurality of sets of the subject identification information UID, the reference genome data identification information GID, the ID indicating the mutation point, and the measured value (one of A, T, G, and C).

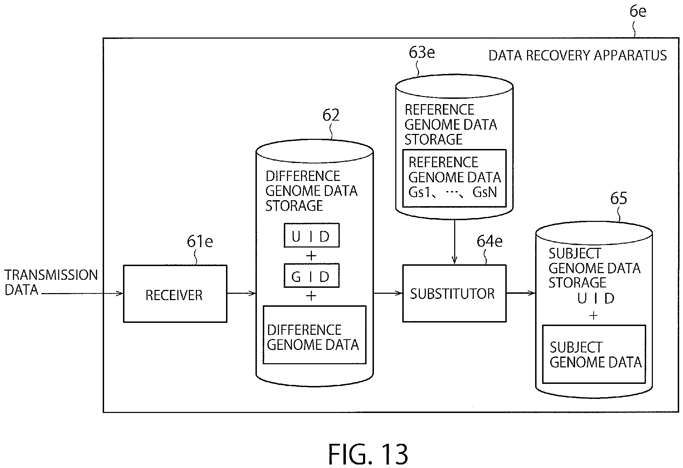

Subsequently, a configuration of the data recovery apparatus 6e in the fourth embodiment will be described with reference to FIG. 13. FIG. 13 is a diagram showing a configuration of the data recovery apparatus 6e in the fourth embodiment. Compared to the configuration of the data recovery apparatus 6 in the first embodiment (see FIG. 4), the receiver 61 is changed to a receiver 61e, the reference genome data storage 63 is changed to a reference genome data storage 63e, and the substitutor 64 is changed to a substitutor 64e in the configuration of the data recovery apparatus 6e in the fourth embodiment. The same reference signs are provided to the same constituent elements as in FIG. 4, and the description will not be repeated.

The receiver 61e receives the transmission data transmitted from the data generation apparatus 4. As a result, the receiver 61e receives the subject identification information UID, the reference genome data identification information GID, and the difference genome data.

The receiver 61e uses the transmission data to associate and store, in the difference genome data storage 62, the subject identification information UID, the reference genome data identification information GID, and the difference genome data.

The N reference genome data Gs1, ..., GsN generated for each attribute class by the genome data generation apparatus 7d are stored in the reference genome data storage 63e.

The substitutor 64e reads the reference genome data specified by the reference genome data identification information from the reference genome data storage 63e and replaces the bases at the positions of the base sequence included in the difference genome data among the bases included in the read reference genome data, with the bases at the positions in the base sequence data of the specific subject. The substitutor 64e associates and stores the substituted subject genome data and the subject identification information UID in the subject genome data storage 65.

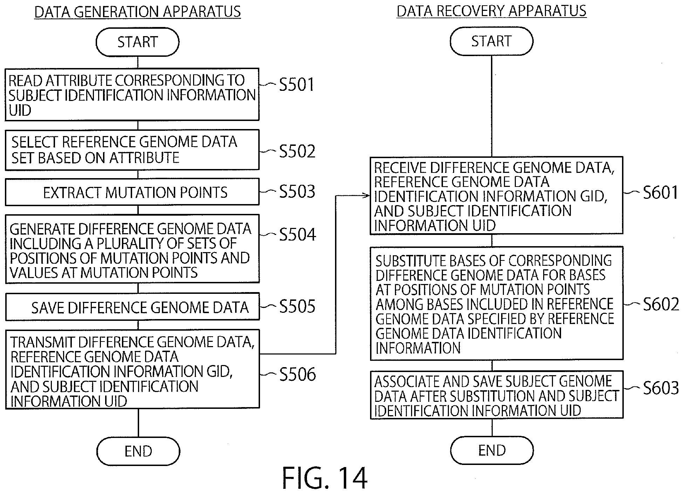

Subsequently, a generation process of the reference genome data in the fourth embodiment will be described with reference to FIG. 14. FIG. 14 is a flow chart showing an example of a generation process of the reference genome data in the fourth embodiment.

(Step S501) The selector 46e reads the attribute corresponding to the subject identification information UID from the attribute storage 45e.

(Step S502) The selector 46e selects the reference genome data based on the attribute read in step S501.

(Step S503) The mutation point extractor 421e compares the reference genome data selected in step S502 and the subject genome data and extracts the mutation points where the base information of the subject genome data at corresponding base sequence positions is different from the base information of the reference genome data.

(Step S504) The difference genome data generator 422 generates the difference genome data including a plurality of sets of the mutation points and the base information (for example, base symbols) at the mutation points in the subject genome data.

(Step S505) The difference genome data generator 422 stores the difference genome data in the difference genome data storage 43 in association with the reference genome data identification information GID for identifying the selected reference genome data and in association with the subject identification information UID.

(Step S506) The transmitter 44 transmits the transmission data including the difference genome data, the reference genome data identification information GID for identifying the selected reference genome data, and the subject identification information UID.

(Step S601) The receiver 61e receives the transmission data including the difference genome data, the reference genome data identification information GID for identifying the selected reference genome data, and the subject identification information UID.

(Step S602) The substitutor 64e substitutes the base information of corresponding difference genome data for the base information at the mutation points among the base information included in the reference genome data specified by the reference genome data identification information GID received by the receiver 61e.

(Step S603) The substitutor 64e associates and saves the substituted and recovered subject genome data and the subject identification information UID in the subject genome data storage 65.

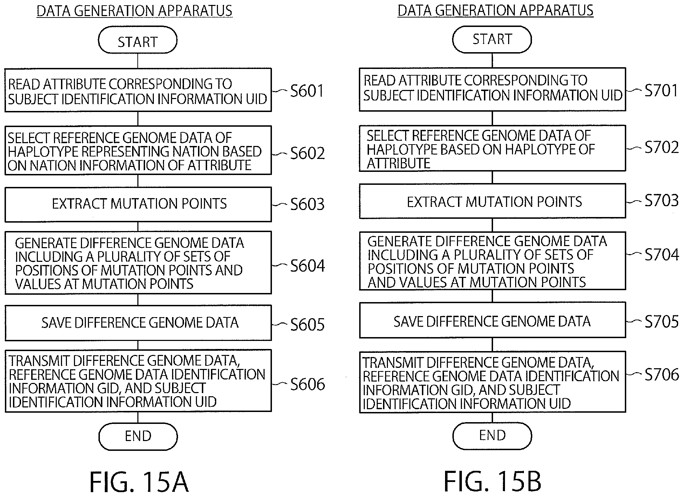

A specific example of the generation process of the reference genome data will be illustrated. FIG. 15A is a flow chart showing a specific example of the generation process of the reference genome data in the fourth embodiment. The attribute class here is a nation, and the reference genome data of the haplotype representing the nation is stored for each nation (attribute class) in the reference genome data storage 41e of FIG. 11. The subject identification information UID and nation information (attribute) indicating the nation that the corresponding subject belongs to are associated and stored as the attribute data of each subject in the attribute storage 45e of FIG. 11. The nation that the subject belongs to is, for example, a nation of the nationality of the subject.

(Step S601) The selector 46e reads the attribute corresponding to the subject identification information UID from the attribute storage 45e. The attribute includes at least the nation information of the subject.

(Step S602) Based on the nation information of the attribute read in step S601, the selector 46e selects the reference genome data of the haplotype representing the nation indicated by the nation information from the reference genome data storage 41e.

(step S603) The mutation point extractor 421e compares the reference genome data selected in step S602 and the subject genome data and extracts the mutation points where the base information of the subject genome data of corresponding base sequence positions is different from the base information of the reference genome data.

(Step S604) The difference genome data generator 422 generates difference genome data including a plurality of sets of the mutation points and the base information (for example, base symbols) at the mutation points in the subject genome data.

(Step S605) The difference genome data generator 422 stores the difference genome data in the difference genome data storage 43 in association with the reference genome data identification information GID for identifying the selected reference genome data and in association with the subject identification information UID.

(Step S606) The transmitter 44 transmits the transmission data including the difference genome data, the reference genome data identification information GID for identifying the selected reference genome data, and the subject identification information UID.

The data recovery apparatus receives the transmission data transmitted in step S606. The operation flow of the data recovery apparatus is the same as the flow chart on the right side of FIG. 14, and the description will not be repeated.

Optimal reference genome data is selected for each nation in the specific example illustrated in FIG. 15A. Therefore, the specific example is suitable in a nation in which main people residing in the nation have a specific haplotype. Examples of the combination of the nation and the people include Japanese people in Japan and Korean people in South Korea and North Korea.

FIG. 15B is a flow chart showing another specific example of the generation process of the reference genome data in the fourth embodiment. The attribute class here is a haplotype, and the reference genome data corresponding to the haplotype is stored for each haplotype (attribute class) in the reference genome data storage 41e of FIG. 11. The subject identification information UID and the corresponding haplotype of the subject are associated and stored as the attribute data of each subject in the attribute storage 45e of FIG. 11.

(Step S701) The selector 46e reads the attribute corresponding to the subject identification information UID from the attribute storage 45e. The attribute includes at least the haplotype of the subject.

(Step S702) Based on the haplotype included in the attribute read in step S701, the selector 46e selects the reference genome data of the haplotype from the reference genome data storage 41e.

(Step S703) The mutation point extractor 421e compares the reference genome data selected in step S702 and the subject genome data and extracts the mutation points where the base information of the subject genome data at corresponding base sequence positions is different from the base information of the reference genome data.

(Step S704) The difference genome data generator 422 generates difference genome data including a plurality of sets of the mutation points and the base information (for example, base symbols) at the mutation points in the subject genome data.

(Step S705) The difference genome data generator 422 stores the difference genome data in the difference genome data storage 43 in association with the reference genome data identification information GID for identifying the selected reference genome data and in association with the subject identification information UID.

(Step S706) The transmitter 44 transmits the transmission data including the difference genome data, the reference genome data identification information GID for identifying the selected reference genome data, and the subject identification information UID.

The data recovery apparatus receives the transmission data transmitted in step S706. The operation flow of the data recovery apparatus is the same as the flow chart on the right side of FIG. 14, and the description will not be repeated.

Optimal genome reference data is selected for each haplotype in the specific example illustrated in FIG. 15B. Therefore, the specific example is suitable for a multi-ethnic nation in which a plurality of types of people with specific haplotypes reside. Examples of the multi-ethnic nation include the United States, Canada, and other immigrant nations, as well as India, Lebanon, and the like where many types of people reside from long ago.

As described, the classifier 76 classifies the plurality of base sequence data into a plurality of attribute classes according to the attributes of the subjects from which the base sequence data are extracted in the fourth embodiment. For each attribute class, the determiner 72 determines the base information with the highest frequency of appearance at each position of SNP by using the plurality of base sequence data classified into the attribute classes. For each attribute class, the generator 73 generates the reference genome data including the base information with the highest frequency of appearance at each position of SNP determined for each attribute class and including the base information at the positions other than the SNP extracted from the plurality of base sequence data.

Therefore, the generator 42 can set the reference genome data of the attribute class that the subject belongs to as the target to be compared with the subject genome data. This can improve the probability of further reducing the number of mutation points. As a result, in addition to the effects of the first embodiment, the probability of reducing the volume of difference data can also be improved. Therefore, the probability of reducing the cost of saving the difference data and the cost of the line can be improved.

The reference genome data of the present embodiment is not personal information, and confidentiality is not necessary. As in the second embodiment, the data does not have to be managed by concealing the data, and the management cost can be reduced.

Although the determiner 72 of the genome data generation apparatuses (7, 7d) according to the embodiments acquires the predetermined SNP positions, the arrangement is not limited to this. The determiner 72 of the genome data generation apparatus 7 may specify the positions of SNP by comparing a plurality of base sequence data of different subjects and determine each position of SNP in the base sequence.

Programs for executing the processes by the data generation apparatuses (4, 4e), the data recovery apparatuses (6, 6e), and the genome data generation apparatuses (7, 7d) of the embodiments may be recorded in a computer-readable recording medium. A computer system may read the programs recorded in the recording medium, and a processor may execute the programs to execute the various processes by the data generation apparatuses, the data recovery apparatuses (6, 6e), and the genome data generation apparatuses (7, 7d) of the embodiments.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the inventions.

### [Reference Signs List]

S1, S5 Genome data transmission systems
1 Sequencing inspection apparatus
2 DNA mapping apparatus
3, 3b Storage devices
4, 4e Data generation apparatuses
41, 41e, 63, 63e, 74 Reference genome data storages
42, 73 Generators
421 Mutation point extractor
422 Difference genome data generator
43, 62 Difference genome data storages
44 Transmitter
45e Attribute storage
46e Selector
5 Communication circuit network
6, 6e Data recovery apparatuses
61, 61e Receivers
64, 64e Substitutors
65 Subject genome data storage
GS3, GS4 Genome data generation systems
7, 7d Genome data generation apparatuses
71 SNP position storage
CON, CON4, CON5 controllers
72 Determiner
721 Analyzer
722 Reference value determiner
75 Sample attribute storage
76 Classifier

## Claims

1. A method of generating reference genome data, comprising:
determining, based on a plurality of base sequence data of different subjects, base information relating to at least one position in a base sequence according to a frequency of appearance of each of a plurality of base information at the position; and
generating reference genome data to associate the determined base information with the position.

2. The method of generating reference genome data according to claim 1, wherein
the at least one position is at least one position of SNP.

3. The method of generating reference genome data according to claim 1 or 2, wherein
the at least one position is each of a plurality of positions of SNP,
the determining comprises determining, for each position of SNP, base information with a highest frequency of appearance among the plurality of base information in the plurality of base sequence data to the base information at the position of SNP, and
the generating comprises generating the reference genome data to associate each determined base information with each corresponding position of SNP.

4. The method of generating reference genome data according to any one of claims 1 to 3, wherein
the plurality of subjects belong to a same attribute class.

5. The method of generating reference genome data according to claim 3 or 4, wherein
the generating comprises generating the reference genome data to further associate base information, at a position other than the position of SNP, extracted from at least one of the plurality of base sequence data with the position other than the position of SNP.

6. The method of generating reference genome data according to any one of claims 1 to 5, further comprising
classifying the plurality of base sequence data into a plurality of attribute classes according to attributes of the subjects from which the plurality of base sequence data is extracted, wherein
the determining comprises determining, for each attribute class, base information with a highest frequency of appearance at each position of SNP in a plurality of base sequence data classified into the attribute class, and
the generating comprises generating, for each attribute class, reference genome data including the base information with the highest frequency of appearance at each position of SNP and including base information, at a position other than the SNP, extracted from the plurality of base sequence data.

7. A method of generating reference genome data comprising:
determining, in a plurality of base sequence data of a plurality of subjects belonging to a same attribute class, base information of at least one position in a base sequence according to a frequency of appearance of each of a plurality of base information at the position; and
generating reference genome data to associate the determined base information with the position.

8. A method of generating difference genome data, comprising
generating difference genome data by comparing subject genome data that is base sequence data of a specific subject with reference genome data set in advance,
the difference genome data including a set of:
position information in a base sequence at a position indicated by which bases are different from each other; and
base information at the position indicated by the position information in the base sequence data of the specific subject.

9. The method of generating difference genome data according to claim 8, further comprising
selecting reference genome data of an attribute to which the specific subject belongs from a plurality of reference genome data provided for a plurality of attribute classes of subjects, wherein
the generating comprises comparing the base sequence data of the specific subject with the reference genome data selected in the selecting.

10. The method of generating difference genome data according to claim 9, further comprising
transmitting the difference genome data and reference genome data identification information for identifying the reference genome data selected in the selecting.

11. The method of generating difference genome data according to claim 10, wherein
the transmitting comprises transmitting subject identification information for identifying the specific subject.

12. The method of generating difference genome data according to any one of claims 9 to 11, further comprising
acquiring subject identification information for identifying the specific subject and reading an attribute corresponding to the acquired subject identification information from a storage device configured to associate and store the subject identification information and the attribute, wherein
the selecting comprises selecting the reference genome data of the read attribute from the plurality of reference genome data, the selected reference genome data being the reference genome data of the attribute to which the specific subject belongs.

13. A data recovery method comprising:
receiving difference genome data including a set of:
a mutation point where a base of subject genome data that is base sequence data of a specific subject and a base of reference genome data set in advance are different from each other; and
a base at the mutation point in the subject genome data; and
substituting the base corresponding to the mutation point in the difference genome data for the base at the mutation point among bases included in the reference genome data.

14. The data recovery method according to claim 13, wherein
the receiving comprises receiving reference genome data identification information for identifying the reference genome data, and
the substituting comprises: reading reference genome data specified by the reference genome data identification information from a storage storing a plurality of reference genome data generated for a plurality of attribute classes of subjects; and replacing, among the bases included in the read reference genome data, the base corresponding to the position of a base sequence included in the difference genome data with the base at the position in the base sequence data of the specific subject.

15. The data recovery method according to claim 13 or 14, wherein
the receiving comprises subject identification information for identifying the specific subject, and
the data recovery method further comprises associating subject genome data obtained by the substituting with the subject identification information and storing them in a storage device.

16. A reference genome data generation apparatus comprising:
a determiner configured to determine, based on a plurality of base sequence data of different subjects, base information relating to at least one position in a base sequence according to a frequency of appearance of each of a plurality of base information at the position; and
a generator configured to generate reference genome data to associate the determined base information with the position.

17. A difference genome data generation apparatus comprising
a generator configured to generate difference genome data by comparing subject genome data that is base sequence data of a specific subject with reference genome data set in advance,
the difference genome data including a set of:
position information in a base sequence at a position indicated by which bases are different from each other; and
base information at the position indicated by the position information in the base sequence data of the specific subject.

18. A data recovery apparatus comprising:
receiving difference genome data including a set of: a position in a base sequence where a base of base sequence data of a specific subject and a base of reference genome data set in advance are different from each other; and a base at the position in the base sequence data of the specific subject; and
replacing, among bases included in the reference genome data, the base corresponding to the position of the base sequence included in the difference genome data with the base at the position in the base sequence data of the specific subject.

19. A program of generating reference genome data which causes a computer to function as:
a determiner configured to determine base information of at least one position of a base sequence in a plurality of base sequence data of different subjects according to a frequency of appearance of the base information at the position; and
a generator configured to generate reference genome data associating the base information determined by the determiner with the position.

20. A program of generating difference genome data which causes a computer to function as:
a generator configured to generate difference genome data by comparing base sequence data of a specific subject and reference genome data set in advance, the difference genome data including a set of position information of base sequences with corresponding bases different from each other and base information at positions indicated by the position information in the base sequence data of the specific subject.

21. A data recovery program which causes a computer to function as:
a receiver configured to receive difference genome data including a set of: a position in a base sequence where a base of base sequence data of a specific subject and a base of reference genome data set in advance are different from each other; and a base at the position in the base sequence data of the specific subject; and
a substitutor configured to replace, among bases included in the reference genome data, the base corresponding to the position of the base sequence included in the difference genome data with the base at the position in the base sequence data of the specific subject.
